# EUROPEAN PATENT APPLICATION

(11) **EP 2 039 736 A1**
(43) Date of publication of application: **25.03.2009**
(21) Application number: 07794085.6
(22) Date of filing: 10.07.2007
(51) Int. Cl.: C09K 11/06, C07F 3/00, C07D 215/58, C07D 215/40

(54) **ELECTROLUMINESCENT MATERIAL CONTAINING AN ORGANIC LUMINESCENT SUBSTANCE**

(30) Priority: 10.07.2006 RU 2006124438
(71) Applicant: Institut Problem Khimicheskoi Fiziki Rossiiskoi AK, Moskovskaya obl., 142432 (RU)
(72) Inventor: YAKUSCHENKO, Igor Konstantinovich, Moskovskaya obl., 142432 (RU); KAPLUNOV, Mikhail Gershovich, Moskovskaya obl., 142432 (RU); KRASNIKOVA, Svetlana Sergeevna, Moskovskaya obl., 142432 (RU)
(74) Representative: Fairbairn, Angus Chisholm
(86) International application number: PCT/RU2007/000379
(87) International publication number: WO 2008/024018

(57) **Abstract**

The invention relates to electroluminescent materials containing organic luminescent substance. The inventive novel electroluminescent material comprises an electron injecting layer, an active luminescent layer based on a luminescent substance, a hole-transport layer and a hole-injecting layer. The material contains metallocomplexes of quinoline-sulfanylamine derivatives in the form of a luminescent substance. The inventive electroluminescent material exhibits the increased time resource, resulting from the high resistance of the active luminescent layer to crystallisation and hydrolysis, and the high heat resistance of the hole-transport layer.

## Description

The invention relates to luminescent materials, in particular to electroluminescent materials containing an organic luminescent substance.

An electroluminescent material (ELM) is known consisting of an electron injecting layer, an active luminescent layer based on a luminescent substance, a hole-transport layer and a hole-injecting layer, the material containing as the luminescent layer an evaporated layer of an organic compound - complexes of aluminum, zinc and some other metals with 8-hydroxyquinoline derivatives [U. Mitschke, P. Bauerle, J. Mater. Chem., 2000, 10, 1471-1507].

The closest to the proposed device in respect of technical essence is an ELM containing tris-(8-hydroxyquinolinate)aluminum as the luminescent layer [C.W. Tang, S.A. Van Slike, Appl. Phys. Letter 51, 913-915 (1987)] (see the figure).

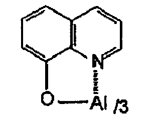

Wherein, a transparent low-resistance layer based on mixed indium oxide and stannum oxide - In₂O₂ - SnO₂ (ITO), is used as the hole-injecting layer (anode), aluminum or magnesium silver alloy as the electron injecting layer (cathode), and N,N'-diphenyl-N,N'-(3-methylphenyl)-1,1'-biphenyl-4,4'-diamine (TPD) as the hole-transport layer.

However, the service life of electroluminescent devices based on such a genus of complexes is often not very long. In addition to a number of other reasons this is related to the properties of these materials: their crystallization in the process of operation of the devices, which changes the homogeneity of the conducting properties of the layer, and also to hydrolysis of the metallocomplexes with traces of water and oxidation with air oxygen and subsequent breakdown thereof [Tokito S. et al., Appl. Phys. Lett. 1997, v. 70, No. 15, p. 1929-1931; Burrows P.E. et al., Appl. Phys. Lett. 1994, v. 65, No. 23, p. 2922-2924]. This also results in a loss of the operating characteristics of this layer.

Furthermore, the service life of the electroluminescent devices is also limited by the low temperature stability of the hole-transport layer materials, which is related to a change of the morphology of the layer upon an increase of the temperature as a result of their low glass-transition temperature (so, for TPD, the glass-transition temperature is 60°C).

The object of the present invention is the creation of an ELM with an increased service life as a result of increased stability of the active luminescent layer in respect of crystallization and hydrolysis, and also increased temperature stability of the hole-transport layer.

This object is resolved in that in accordance with the invention an electroluminiscent material consisting of an electron injecting layer, an active luminescent layer based on a luminescent substance, a hole-transport layer and a hole-injecting layer, contains metallocomplexes with ligands based on 8-aminoquinoline derivatives of general formula (I): 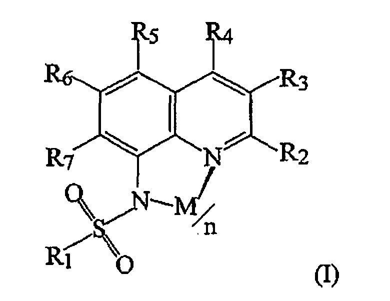 wherein:
M=Zn, Cd, Be (n=2); Al, Ga (n=3);
group R₁ may be selected from the series:
   - an alkyl group consisting of 1-4 carbon atoms, straight or branched;
   - trifluoromethyl, pentafluoroethyl groups;
   - mono- or polyalkyl substituted phenyl group in which alkyl substituents consist of 1-4 carbon atoms and are straight or branched;
   - mono- or trifluoromethyl substituted phenyl group;
   - mono- or polyhalo substituted phenyl group in which halogen atoms are fluorine, chlorine, bromine, iodine;
   - mono- or polyalkoxy substituted phenyl group in which alkoxy substituents consist of 1-4 carbon atoms and are straight or branched;
   - ethylene dioxy substituted phenyl group;
   - dimethyl- or diethylamino substituted phenyl group;
   - mono- or polycyano substituted phenyl group;
   - 1- or 2-naphthyl group;
   - 2-, 3- or 4-pyridyl group;
groups R₂-R₇ may be identical or different and independently of each other may be selected from the series:
   - alkyl group with 1-8 carbon atoms which is both straight and branched or cyclic;
   - trifluoromethyl, pentafluoroethyl group;
   - phenyl, mono- or polyalkyl substituted phenyl group in which alkyl substituents consist of 1-4 carbon atoms and are straight or branched;
   - mono- or polyalkoxy substituted phenyl group in which alkoxy substituents consist of 1-4 carbon atoms and are straight or branched;
   - mono- or polydimethyl or diethylamino substituted phenyl group;
   - mono- or polycyano substituted phenyl group;
   - alkoxy group in which alkyl substituents consist of 1-4 carbon atoms and are straight or branched;
   - dimethyl-, diethylamino group;
   - halogenyl group (fluorine, chlorine, bromine, iodine),
   - cyano group;
   - methoxy-, ethoxycarbonyl group;
   - phenoxyl group;
   - acetyl, benzoyl groups;
   - 2-, 3-, 4-pyridyl group.

Zinc complexes of 8-(methylsulfanilamino)quinoline (II) and 8-(3,5-difluorophenylsulfanilamino)quinoline (III) may be used as examples of substances of formula (I):

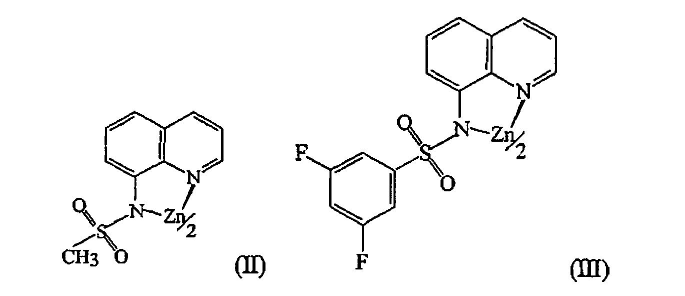

In sulfanylamino derivatives of quinoline, a hydrogen atom bound to a nitrogen atom, in respect of its acidity, is comparable to a corresponding hydrogen atom of a phenol hydroxyl. This makes it possible to obtain stable salts (metallocomplexes) with metal ions.

Bulk substituents at a nitrogen atom in a metallocomplex should make rapid crystallization of the latter in the electron transport layer difficult during operation of the device, which may improve the service life in comparison with a device wherein metallocomplexes of 8-hydroxyquinoline are used.

The bulk substituents of alkyl- or arylsulfanyl fragments in 8-aminoquinoline screen the approach of a water molecule to a nitrogen-metal bond, which makes hydrolysis of the metallocomplex difficult as compared with such on the basis of 8-hydroxyquinoline. This should also have a positive effect on the service life of the device.

The stated object is also resolved in that the material preferably comprises as the hole-transport layer a mixture of triphenylamine oligomers with the general formula 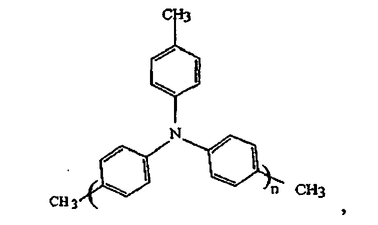 wherein n=8-9, with the molecular-weight distribution: Mn=2332, Mw=3586, having a high glass-transition temperature of 185°C, which provides a retention of the morphology of the hole-transport layer even at higher temperatures [Jakushchenko I.K., Kaplunov M.G., Shamaev S.N., Efimov O.N., Nikolaeva G.V., Belov M.Ju., Marchenko E.P., Skvortsov A.G., Voronina V.A. "Method of preparing mixture of oligotriphenyl amines, method of preparing 3-(4-biphenyl)-4-(4-tertbutylphenyl)-5-(4-dimethylaminophenyl)-1,2,4-triazole and an electroluminescent device" Patent RU No. 2131411 of 10 June 1999].

The invention is illustrated by the following examples.

### Example 1

### Synthesis of 8-(methanesulfanilamino)quinoline (IV)

Syntheses are carried out in accordance with a known method ["Methods for preparation of chemical reagents and preparations," No. 4-5, Moscow, IREA, 1962, pages 67-69].

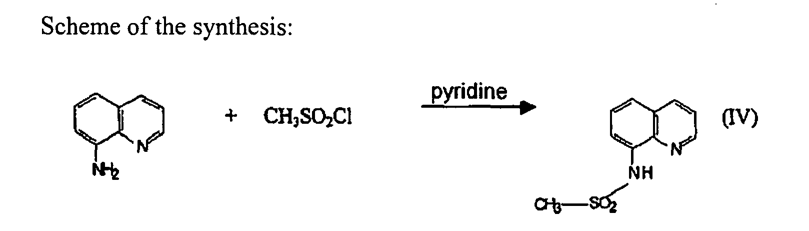

A solution of 2.8 g (0.02 M) of methanesulfochloride in 3.5 ml of anhydrous tetrahydrofurane was added to a solution of 2.88 g (0.02 M) of 8-aminoquinoline in 10 ml of anhydrous pyridine while cooling to 3-5°C. The length of adding was 15-20 min. Then the mixture was stirred at the same temperature for 30 min, further for 1 hour while boiling the reaction mixture. After that the solvent was driven off, the residue cooled to room temperature and treated with 75 ml of water. The formed residue was filtered, washed with 20 ml of water, dried in air. An unpurified product (IV) in an amount of 4.54 g was obtained. The compound (IV) was recrystallized from benzol (with the subsequent addition of hexane). A pure substance in an amount of 3.93 g was obtained.
Melting point 146.5-147°C. Yield - 88.5% of the theoretical value.
Element analysis. Found, %: C 55.74; H 4.78; N 12.15; S 14.34.
Molecular formula - C₁₀H₁₀N₂O₂S. Calculated, %: C 54.04; H 4.54; N 12.60; S 14.34.
PMR-spectrum δ(ppm) 3.15 (3H, CH₃-, s.), 7.59-7.63 (H₃ d.d.), 7.64-7.68 (H₆ d.d.), 7.73-7.75 (H₇ d.), 7.75-7.77 (H₅ d.), 8.44-8.46 (H₄ d.), 8.94-8.96 (H₂ d.), 9.39 (NH, s.).
Mass-spectrum: m/e (I/Imax, %): 222 (M, 43), 143 (100), 116 (75), 89 (31), 63 (22), 39 (15).

### Example 2

### Synthesis of 8-(3,5-difluorophenylsulfanilamino)quinoline (V)

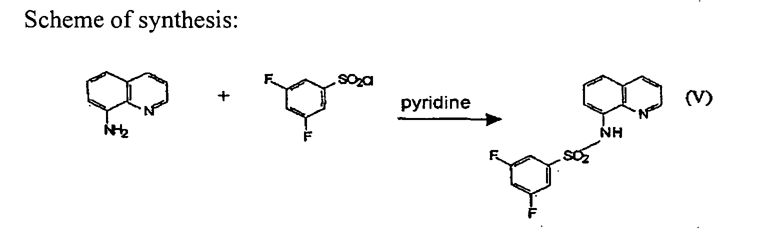

A solution of 3.4 g (0.016 M) of 3,5-difluorophenylsulfochloride in 10 ml of tetrahydrofurane was added to a solution of 2.02 g (0.014 M) of 8-aminoquinoline in 5 ml of anhydrous pyridine while cooling to 3-5°C. The length of adding was 10-15 min. The mixture was stirred at the same temperature for 30 min, further for 1 hour at room temperature and more hour while boiling the reaction mixture. After that the mixture was cooled and treated with 250 ml of cold water. The obtained residue was filtered, washed with water, dried. The obtained unpurified product (4.75 g) was dissolved upon heating in benzol, the insoluble admixtures were filtered off the solution, hexane was added to the filtrate. The residue of the compound (V) thereby obtained was filtered, washed with hexane, dried. The substance VI in an amount of 3.89 g was obtained. The melting point was 119-119.5°C. The yield - 87% of the theoretical value.

Element analysis. Foun d, %: C 54.00; H 4.21; N 8.70; S 10.37. Molecular formula- C₁₅H₁₀F₂N₂O₂S. Calculated, %: C 56.25; H 3.15; N 8.75; S 10.01.

PMR-spectrum δ(ppm) 7.53-7.58 (1H from Ph, H₃, m.), 7.58-7.71 (H₆ d.d.), 7.63-7.70 (2H, Ph, m.), 7.69-7.72 (H₇, d.), 7.73-7.76 (H₅, d.), 8.38-8.40 (H₄, d.), 8.85-8.87 (H₂, d.), 10.5 (NH, s.).

Mass spectrum: m/e (I/Imax, %): 320 (M, 35), 256 (40), 143 (100), 116 (78), 89 (31), 63 (27), 39 (12).

### Example 3

### Synthesis of bis-((8-methanesulfanilamino)quinolinate)zinc (II)

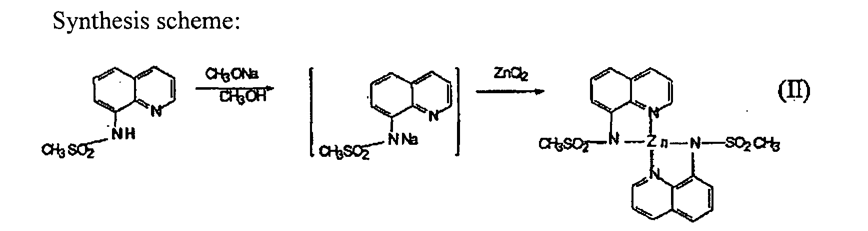

2.22 g (0.01 M) of 8-(methanesulfanilamino)quinoline (IV) were suspended in 25 ml of anhydrous methanol. A solution of sodium methylate, obtained by dissolving 0.23 g (0.01 M) of sodium in 6 ml of methanol, was added to the aforesaid mixture at room temperature. Wherein, a residue of sodium salt of compound (IV) was formed. The mixture was stirred for 30 min at the same temperature, after which a solution of 0.68 g (0.005 M) of anhydrous zinc chloride in the form of a solution thereof in 5 ml of anhydrous methanol was added drop-by-drop. Then the mixture was stirred for 2 hours while heating to 50-55°C. After cooling, a white color residue was filtered, washed sequentially with water, methanol. Dried in a vacuum above P₂O₅. Compound (II) in an amount of 2.41 g was obtained. The yield was 95.1% of the theoretical value. For additional purification the substance was recrystallized from tetrahydrofurane. The substance does not melt at a temperature below 380°C.
Element analysis. Found, %: C 47.57; H 3.99; N 10.36; S 11.46; Zn 12.86. Molecular formula - C₂₀H₁₈N₄O₄S₂Zn. Calculated, %: C 47.30; H 3.57; N 11.03; S 12.63; Zn 12.87.
UV-spectrum: 242, 265, 382 nm (absorption, applied on quartz), 500 nm (photoluminescence, powder, λ_{excit}=380 nm)
IR-spectrum (table KBr): 3094, 3067, 3016, 3005, 2928, 2850, 1604, 1584, 1467, 1428, 1418, 1392, 1385, 1331, 1321, 1285, 1273, 1248, 1207, 1196, 1130, 1078, 1044, 996, 977, 956, 881, 829, 804, 790, 768, 734, 663, 640, 590, 540, 552, 526, 517, 444.

The presence of valence-oscillation bands C-H in the field 3000-3100 cm⁻¹ and the double-bond oscillation bands C=C in the range 1500-1600 cm⁻¹ confirms the presence of aromatic rings and system of conjugated carbon-carbon bonds.

### Example 4

### Synthesis of bis-[8-(3.5-difluorophenylsulfanilamino)quinolinate] zinc (III)

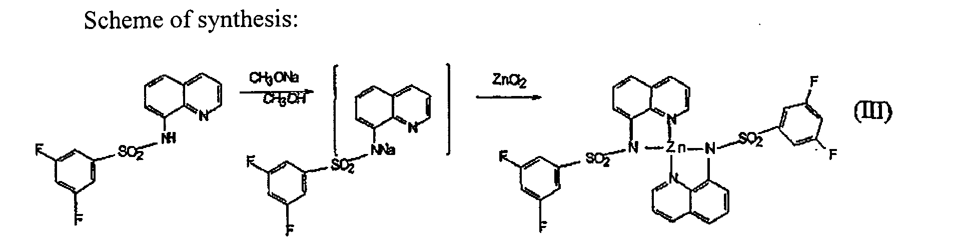

1.92 g (0.06 M) of 8-(3.5-difluorophenylsulfanilamino)quinoline (V) was suspended in 25 ml of dry methanol. A solution of sodium methylate obtained by dissolving 0.14 g (0.06 M) of sodium in 6 ml of methanol was added to the aforesaid suspension at room temperature. The suspension was dissolved and the mixture was stirred for 30 min while being heated to 35-40°C. Then a solution of 0.41 g (0.003 M) of anhydrous zinc chloride in 10 ml of methanol was added to the mixture drop-by-drop. A white residue was formed. It was stirred at 35-40°C for 1 more hour, then cooled to room temperature, filtered, washed with water, methanol, dried. Compound (III) in an amount of 2.01 g was obtained. The melting point was 308-309°C. The yield was 95% of the theoretical value. For additional purification the substance was recrystallized from tetrahydrofurane.
Element analysis. Found, %: C 52.00; H 3.32; N 7.65; S 9.10. Molecular formula - C₃₀H₁₈F₄N₄O₄S₂Zn. Calculated, %: C 51.19; H 2.58; N 7.96; S 9.10.
UV-spectrum: 265, 370 nm (absorption, applied on quartz), 465 nm (photoluminescence, powder, λ_{excit}=380 nm)
IR-spectrum (table KBr): 3075, 3043, 3924, 2853, 1606, 1587, 1504, 1468, 1439, 1384, 1327, 1294, 1276, 1245, 1209, 1195, 1144, 1123, 1084, 1047, 987, 971, 894, 971, 894, 859, 825, 796, 788, 759, 750, 678, 665, 636, 611, 596, 578, 538, 511.

The presence of valence-oscillation bands C-H in the field 3000-3100 cm⁻¹ and the double-bond oscillation bands C=C in the range 1500-1600 cm⁻¹ confirms the presence of aromatic rings and system of conjugated carbon-carbon bonds.

### Example 5

### Electroluminescent properties of complexes II and III

In order to produce an electroluminescent device with an ITO/HTL/EML/A1 structure, where ITO is a hole-injecting layer, HTL - a hole-transport layer, EML - an electroluminescent layer and A1 - an electron injecting layer, a glass substrate is used that is coated with a transparent layer of mixed indium and tin oxide with a resistance of 20-25 ohm/square. A hole-transport layer consisting of PTA is applied onto the substrate. Wherein the PTA is applied by the centrifugation method from a solution in toluene. The thickness of the hole-transport layer is 0.05-0.1 micron. Then by evaporation of complex II or complex III, in a vacuum at a temperature of about 350°C and base pressure of 5×10⁻⁶ mm Hg an active electroluminescent layer is applied with a thickness of 0.02-0.05 µm. The sample is placed in a vacuum installation VUP-4, pumped out in a dynamic mode to a vacuum of 5×10⁻⁶ mm Hg and a metal electrode is sprayed by evaporation of aluminum. The thickness of the metal electrode is about 0.1 µm. The area of the glowing surface is 4-5 mm². The obtained electroluminescent device radiates blue-green light upon the application of a direct voltage. A device containing II has the following parameters: brightness 140 cd/m² is achieved at a voltage of 19 V and current density 1.5 mA/cm² (effectiveness 9 cd/A). A device containing III has the following parameters: brightness 24 cd/m² is achieved at a voltage of 20 V and current density of 19 mA/cm² (effectiveness 1.2 cd/A).

So, in the instant invention an electroluminescent material is created with an increased service life due to an increased stability of the active luminescent layer in respect to crystallization and hydrolysis, and also an enhanced temperature stability of the hole-transport layer.

## Claims

1. An electroluminiscent material consisting of an electron injecting layer, an active luminescent layer based on a luminescent substance, a hole-transport layer and a hole-injecting layer, **characterized in that** it contains as the luminescent substance metallocomplexes with ligands based on 8-aminoquinoline derivatives of general formula (I): 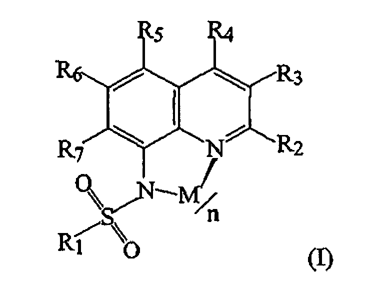 wherein:
M=Zn, Cd, Be (n=2); Al, Ga (n=3);
group R₁ may be selected from the series:
- an alkyl group consisting of 1-4 carbon atoms, straight or branched;
- trifluoromethyl, pentafluoroethyl groups;
- mono- or polyalkyl substituted phenyl group in which alkyl substituents consist of 1-4 carbon atoms and are straight or branched;
- mono- or trifluoromethyl substituted phenyl group;
- mono- or polyhalo substituted phenyl group in which halogen atoms are fluorine, chlorine, bromine, iodine;
- mono- or polyalkoxy substituted phenyl group in which alkoxy substituents consist of 1-4 carbon atoms and is straight or branched;
- ethylene dioxy substituted phenyl group;
- dimethyl- or diethylamino substituted phenyl group;
- mono- or polycyano substituted phenyl group;
- 1- or 2-naphthyl group;
- 2-, 3- or 4-pyridyl group;
groups R₂-R₇ may be identical or different and independently of each other may be selected from the series:
- alkyl group with 1-8 carbon atoms, which is both straight and branched or cyclic;
- trifluoromethyl, pentafluoroethyl group;
- phenyl, mono- or polyalkyl substituted phenyl group in which alkyl substituents consist of 1-4 carbon atoms and are straight or branched;
- mono- or polyalkoxy substituted phenyl group in which alkoxy substituents consist of 1-4 carbon atoms and are straight or branched;
- mono- or polydimethyl- or diethylamino substituted phenyl group;
- mono- or polycyano substituted phenyl group;
- alkoxy group in which alkyl substituents consist of 1-4 carbon atoms and is straight or branched;
- dimethyl-, diethylamino group;
- halogenyl group (fluorine, chlorine, bromine, iodine),
- cyano group;
- methoxy-, ethoxycarbonyl group;
- phenoxyl group;
- acetyl, benzoyl groups;
- 2-, 3-, 4-pyridyl group.

2. The electroluminescent material according to claim 1, **characterized in that** it comprises as the luminescent substance a zinc complex 8-(methylsulfanilamino)quinoline (II):

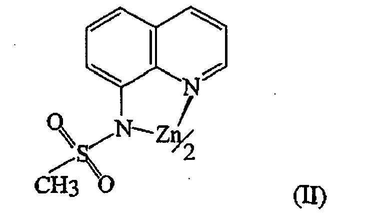

3. The electroluminescent material according to claim 1, **characterized in that** it comprises as the luminescent substance a zinc complex 8-(3.5-difluorophenylsulfanilamino)quinoline (III):

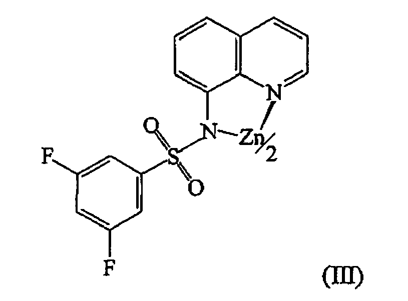

4. The electroluminescent material according to claim 1, **characterized in that** it comprises as the hole-transport layer a mixture of triphenylamine oligomers with the general formula 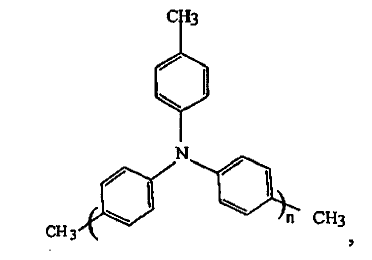 wherein n=8-9, with the molecular-weight distribution: Mn=2332, Mw=3586.
